# EUROPEAN PATENT SPECIFICATION

(11) **EP 2 600 881 B1**
(45) Date of publication and mention of the grant of the patent: **26.01.2022**
(21) Application number: 11754543.4
(22) Date of filing: 03.08.2011
(51) Int. Cl.: A61K 47/02, A61K 36/49, A61K 9/00, A61P 17/00, A61P 19/10, A61K 47/06, A61K 9/06, A61K 9/08, A61K 9/10, A61K 9/48, A61P 17/02, A61P 29/00, A61P 31/04, A61P 31/12

(54) **NATURAL MEDICINAL COMPOUND**
NATÜRLICHE MEDIZINISCHE VERBINDUNG
COMPOSÉ MÉDICINAL NATUREL

(30) Priority: 05.08.2010 IT CR20100025
(43) Date of publication of application: 12.06.2013
(73) Proprietor: LT NATURAL GROUP S.R.L., 26011 Casabuttano ed Uniti (CR) (IT)
(72) Inventor: GRAZIANI, Rodolfo, I-47023 Cesena (IT); MERCORELLA, Giovanni, I-40050 Monteveglio (BO) (IT); ROSSI, Pierenrico, I-47023 Cesena (FC) (IT); BIGNETTI, Guido, I-25082 Botticino Sera (BS) (IT); PIGNACCA, Michele, I-43039 Salsomaggiore Terme (PR) (IT)
(74) Representative: Marcio', Paola
(86) International application number: PCT/IT2011/000288
(87) International publication number: WO 2012/017471

(56) References cited:
- EP-A2- 0 481 396
- WO-A1-01/91589
- WO-A1-2005/079741
- DE-A1- 1 492 909
- QUAVE CASSANDRA L ET AL: "Quorum sensing inhibitors of Staphylococcus aureus from Italian medicinal plants.", PLANTA MEDICA JAN 2011 LNKD- PUBMED:20645243, vol. 77, no. 2, 19 July 2010 (2010-07-19), pages 188-195, XP002637765, ISSN: 1439-0221
- BUDRIESI ROBERTA ET AL: "Stop Fitan: antispasmodic effect of natural extract of chestnut wood in guinea pig ileum and proximal colon smooth muscle.", JOURNAL OF MEDICINAL FOOD OCT 2010 LNKD- PUBMED:20626243, vol. 13, no. 5, 13 July 2010 (2010-07-13), pages 1104-1110, XP002637766, ISSN: 1557-7600
- LUPINI C ET AL: "In vitro antiviral activity of chestnut and quebracho woods extracts against avian reovirus and metapneumovirus", RESEARCH IN VETERINARY SCIENCE, vol. 87, no. 3, December 2009 (2009-12), pages 482-487, XP002637767, ISSN: 0034-5288

## Description

This invention refers to the sector of drugs intended for use both on humans and animals, and it particularly concerns a natural medicinal compound based on a vegetal active ingredient.

The invention belongs to the field of medicinal compounds used for treating:
- inflammation-related diseases;
- viral diseases;
- degenerative diseases such as osteoporosis;
- diseases caused by bacteria or fungi;
- dermatitis and psoriasis;
- lesions, ulcers, wounds, burns, insect bites.

Currently, cortisone-based drugs or common NSAIDs (non-steroidal anti-inflammatory drugs) that can be taken in various forms are prescribed for the treatment of inflammatory diseases: for topical use in salves, gel, ointments and dermatological creams, or for oral use in tablets or capsules.

Synthetic drugs are usually prescribed for the treatment of viral diseases, or said diseases are preventively countered by vaccines, while various supplements (based on calcium, magnesium, vitamins, etc.) are taken for degenerative diseases, such as osteoporosis.

Antibiotics and drugs typical of conventional medicine are currently prescribed for the treatment of diseases deriving from bacteria or fungi, of dermatitis or for the regeneration of tissues that have suffered lesions and burns.

Disadvantageously all these treatments have contraindications.

NSAIDs applied on the skin can encourage phenomena such as photosensitisation, while cortisone-based drugs can facilitate the development of mycosis and alter immune defences. Furthermore, uninterrupted consumption both of NSAIDs and of cortisone-based drugs can cause gastric damage and ulcerous lesions in mucous tissue and, more seriously, adrenal insufficiency.

Disadvantageously vaccines contain toxic substances: heavy metals, carcinogens, strong antibiotics, substances that hyperexcite the immune system of the patient.

Vaccines can, in fact, cause severely disabling diseases in the vaccined person, and the principal diseases that can be triggered by vaccines include: ischemia, intoxication, inflammation, genetic mutation of mitochondrial DNA, mutations transmitted from the mother to the foetus, immunodepression, etc.

Supplements are recommended for cases in which the body presents a deficiency of certain nutrients: therefore, they do not possess actual curative properties, but are necessary to supplement a normal diet by completing it. Hence, they have a relatively bland effect, and are not very effective treatment for serious diseases.

In some cases, drugs based on natural vegetal extracts can be used as an alternative to conventional drugs or combined with synthetic active ingredients.

The use of polyphenols and, particularly, of tannin of vegetal origin is known to either fight or prevent the development of certain diseases.

Tannins are polyphenol compounds that are common in vegetal cells, and their content is high in the bark of plants, such as oak, chestnut, spruce and acacia.

Tannins are commonly used in the leather and wine manufacturing industries, but they also have several pharmacological applications: administered either topically or orally, they have an antibacterial and antifungal effect, administered only orally they have an anti-diarrhoeic effect, administered only topically they have a vasoconstrictive effect; they are used for regeneration of tissues with wounds and burns and, lastly, they are used for the treatment of dermatitis.

Document DE 14 92 909 A1 discloses the use of tannin extracted from chestnut wood (castanea sativa) in aqueous solution as dietary supplement for promoting the development of industrially bred animals. Said dietary supplement nevertheless contains a very low percentage of tannin, between 0,05 and 3% in weight.

Tannin-based compounds, which are currently used only topically in pharmacology, include a very low percentage of tannin, below 5% of their weight, to avoid problems of absorption, difficult penetration of the active ingredient and, hence, wastage of the drug, and to avoid the development of unaesthetic skin patches on the application surface.

Disadvantageously the curative effect of salves or creams with such a low percentage of tannin is rather limited.

These disadvantages resulting from the application of tannin-based compounds are a strong reason for prejudice on the part of the average technician who is, therefore, reluctant to study new formulations of medicinal compounds containing tannin and, especially, new potential applications of the same for the treatment of diseases that are commonly treated with other drugs.

This invention has the principal scope of providing a medicinal compound entirely based on natural and vegetal active ingredients for the treatment of all the aforementioned diseases, without contraindications.

Again, scope of the invention is to create a medicinal compound for easy and rapid application, with a good degree of absorption and excellent tolerability for the body that uses it.

With these objectives, the inventors surprisingly discovered that, for diseases caused by bacteria or fungi, or when there are lesions, burns, insect bites or degenerative diseases, increasing the percentage of tannin and using, instead of pure tannin, a natural extract containing a mixture of tannin, polyphenols and other nutritional elements and adding appropriate carrier means, produces better curative efficacy along with good absorption of the product.

Even more surprising the discovery that of all tannins derived from the bark of plants, the most effective is chestnut tannin (*Castanea sativa)*, which is contained in a remarkable percentage in natural chestnut extract (NCE), which also has a powerful antiviral, anti-inflammatory and antiaging action.

The aforementioned aims are achieved with a natural medicinal compound containing a tannin-based vegetal active ingredient combined with carriers and with a synergic effect, characterized in that said vegetal active ingredient is natural chestnut extract (*Castanea sativa)*, with tannin as the lightest constituent substance, wherein said natural chestnut extract is obtained by hydrolysis of chestnut wood (*Castanea sativa)* without the addition of foreign or synthetic substances, wherein the percentage of tannin contained in the natural chestnut extract is about 77%, wherein the natural chestnut extract is present in a weight rate between 5% and 50%, and said carriers are water-based and comprise alkaline substances.

In a further embodiment said compound is a medicinal suspension wherein the natural chestnut extract is present in a weight rate between 5% and 50% and said carrier means are water-based and comprise cross-linkers.

Compared to current tannin-based compounds that are topically used with antibacterial, antifungal and vasoconstrictive effects, with a regenerating effect on tissues presenting lesions, burns or dermatitis, the drug according to the present invention is highly effective, easy and quick to absorb, free of undesirable side-effects, such as formation of scabs or of particular skin patches at the site of application.

Even more advantageously, the medicinal compound based on natural chestnut extract according to the present invention, appropriately dosed and conveyed, forms creams, emulsions, suspensions, solutions, etc. suitable ideal for treating the most diverse diseases, ranging from viral diseases, such as herpes simplex or herpes zoster, to psoriasis, or soft oedematous cellulite.

The use according to the present invention of natural chestnut extract to form capsules, for instance, for treating osteoporosis, or for normalising cholesterol levels is of even greater advantage.

These and other aims, advantages and characteristics are specified in the description below of some preferred embodiments, herein listed only as a non-limiting example.

In all the following applicative examples, the basic active ingredient of the compounds is natural chestnut extract, commonly known as NCE, wherein the most precious part is chestnut tannin (*Castanea sativa)*, which is present as the highest constituent substance.

Said natural chestnut extract is obtained by hydrolysis of chestnut wood (*Castanea sativa)* without the addition of foreign or synthetic substances.

During the extraction process, chestnut trunks are mechanically cleaned of the bark and branches and crushed to pieces, which are later washed and channelled to an autoclave for processing at high temperatures and pressures.

The aqueous solution obtained contains a mixture of all polyphenols and other nutritional elements typical of natural chestnut extract: hydrosoluble polyphenols, simple sugars, lignin, mineral salts, cellulose and hemicellulose.

Natural chestnut extract powder obtained through a known technological process starting from the aqueous solution does not contain only pure tannin, but tannin combined with other natural substances deriving from the extraction process.

Natural chestnut extract powders available on the market have a pure tannin content between 60% and 99%.

The following is, for instance, the technical fact sheet of a natural chestnut extract wherein the pure chestnut's tannin content is 77%. This fact sheet underscores the specific chemical and physical parameters thereof, by which the best experimental results have been achieved, and the percentages of the constituent elements thereof.

| **PARAMETER** | **UNITS** | **MEAN VALUE** |
|---|---|---|
| TANNIN | % | 77 |
| NOT TANNIN | % | 16 |
| T/Nt ratio | % | 4.5 |
| HUMIDITY | % | 7 |
| DRY SUBSTANCE | % | 92 |
| ASH | % | 1.6 |
| PH | % | 3.6 |
| ROUGH FIBER | % | <0,5 |
| NDF | % | 2 |
| ADF | % | 1.2 |
| ADL | % | 0.89 |
| RAW PROTEINS (Nx065) | % | 3.74 |
| TOT NITROGEN SUBSTANCES | % | 0.8 |
| RAW LIPIDS | % | 0.58 |
| MINERALS | % | 1.5 |
| TOTAL GLUCIDS, | % | 10 |
| with reducing agents | % | 4.5 |
| SULPHUR | mg/Kg | 1800 |
| PHOSPHORUS | mg/Kg | 780 |
| CALCIUM | mg/Kg | 1300 |
| MAGNESIUM | mg/Kg | 1000 |
| POTASSIUM | mg/Kg | 1900 |
| SODIUM | mg/Kg | 1400 |
| IRON | mg/Kg | 80 |
| MANGANESE | mg/Kg | 190 |
| COPPER | mg/Kg | <0.1 |
| ZINC | mg/Kg | 4 |
| LEAD | mg/Kg | <1 |
| CADMIUM | mg/Kg | <0.2 |

The aforementioned natural chestnut extract with 77% tannin content was used in the applicative examples below.

### Example No. 1

According to a preferred embodiment to produce the medicinal compound according to the present invention, the water-based solution used to produce it contains 30% weight rate of natural chestnut extract.

Synergic alkaline substances, such as NOH, KOH or amine, have been added to water to make tannin soluble.

Tannin is, in fact, an acid substance that is salified and dissolved by the addition of alkali. Furthermore, the solution can be thickened with known techniques to obtain the desired fluidity.

Advantageously, when the solution is applied, for instance, on a wound, the salt penetrates into the skin which has an acidic pH, the tannin contained in the natural chestnut extract is hydrolysed and restored to the acid form, which is the biologically active one, and encourages cicatrisation.

For instance, excipients, which act synergically and amplify its beneficial effect, can be added to this basic solution. Said excipients can be for example: zinc oxide, dry or fluid aloe extracts, calendula or other, essential oils such as *melaleuca alternifolia* (tea tree) or rosemary.

### Example No. 2

According to another embodiment not part of the invention, natural chestnut extract powder can be encapsulated.

Specifically, capsules containing 500 mg of natural chestnut extract powder are advantageously used to regularise the digestive system, control intestinal bacterial flora, regulate blood cholesterol levels, encourage the circulatory system, and treat viral hepatitis type C.

Instead, capsules containing 200 mg of natural chestnut extract powder, 100 mg of gentian, 100 mg of equisetum and 100 mg of impalpable calcium carbonate are advantageously used to treat osteoporosis.

Tannin contained in capsules dissociates the calcium carbonate present in said capsules, but also the calcium carbonate ingested by the patient with food, releasing positive Ca⁺⁺ ions. This dissociation increases the capacity to absorb calcium in the ionic form and makes it bioavailable and easily absorbable by the body and by the skeletal system with subsequent beneficial effects.

### Laboratory Test No. 1

The efficacy, *in vitro,* of some medicinal compounds based on natural chestnut extract against bacterial species of avian origin has been tested.

The test made use of bacterial strains from the IZSLER collection. A concentration of 10⁶ cfu/ml was prepared.

The bacterial strains were planted in dishes containing culture media.

For liquid products, disks soaked with 50 µL of product were positioned on the culture media. Solid products were placed in wells cut in the centre of culture media.

The diameter of the inhibition halo including the diameter of the disk or of the well (12.7 mm) was read after 24 hours of incubation at 37°C, and results were recorded in the table below.

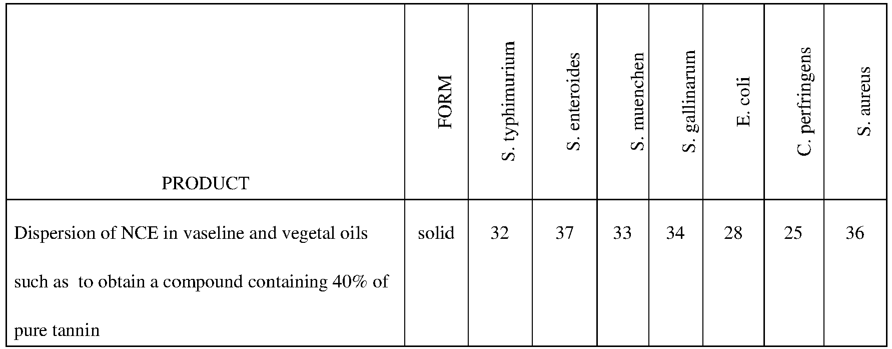

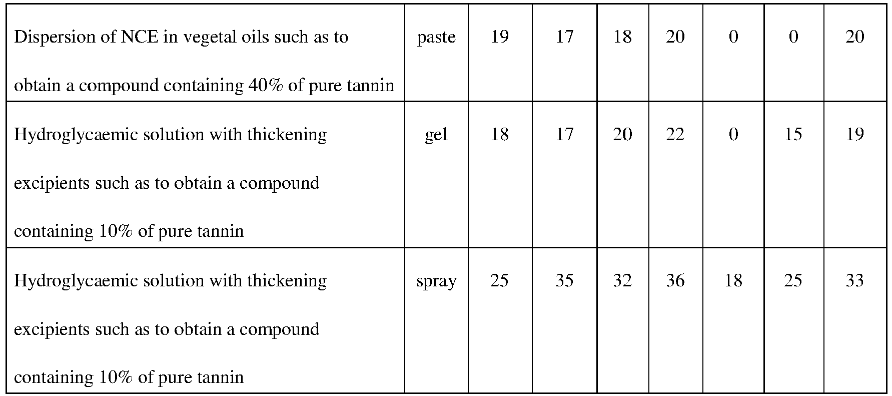

## Claims

1. Natural medicinal compound containing a tannin based vegetal active ingredient combined with carriers and with a synergic effect, **characterized in that** said vegetal active ingredient is natural chestnut extract (*Castanea sativa),* with tannin as the highest constituent substance, wherein said natural chestnut extract is obtained by hydrolysis of chestnut wood (*Castanea sativa)* without the addition of foreign or synthetic substances, wherein the tannin content in the natural chestnut extract is approximately 77%, wherein said natural chestnut extract is contained in a weight rate between 5% and 50% and said carrier means are water-based and comprise alkaline substances.

2. Medicinal suspension, **characterized by** the fact that it comprises the medicinal compound according to claim 1, wherein said natural chestnut extract is contained in a weight rate between 5% and 50% and said carrier means are water-based and include cross-linkers.

3. The medicinal compound according to claim 1, for use in treating inflammations, viral diseases, bacterial diseases, wounds, burns and osteoporosis.

## Patentansprüche

1. Natürliche medizinische Verbindung, die einen pflanzlichen Wirkstoff auf Tanninbasis, kombiniert mit Trägern und mit einer synergetischen Wirkung, enthält, **dadurch gekennzeichnet, dass** der besagte pflanzliche Wirkstoff ein natürlicher Kastanienextrakt (*Castanea sativa*) ist, mit Tannin als höchstem Bestandteil, wobei der besagte natürliche Kastanienextrakt durch Hydrolyse von Kastanienholz (*Castanea sativa*) ohne Zusatz von fremden oder synthetischen Substanzen erhalten wird, wobei der Tanningehalt in dem natürlichen Kastanienextrakt ungefähr 77% beträgt, wobei der besagte natürliche Kastanienextrakt in einem Gewichtsanteil zwischen 5% und 50% enthalten ist und die besagten Trägermittel auf Wasserbasis sind und alkalische Substanzen umfassen.

2. Medizinische Suspension, **dadurch gekennzeichnet, dass** sie die medizinische Verbindung gemäß Anspruch 1 enthält, wobei der besagte natürliche Kastanienextrakt in einem Gewichtsanteil zwischen 5% und 50% enthalten ist und die besagten Trägermittel auf Wasserbasis sind und Vernetzer enthalten.

3. Die medizinische Verbindung gemäß Anspruch 1, zur Verwendung bei der Behandlung von Entzündungen, Viruserkrankungen, bakteriellen Erkrankungen, Wunden, Verbrennungen und Osteoporose.

## Revendications

1. Composé médicinal naturel contenant un ingrédient actif végétal à base de tanin combiné à des supports et ayant un effet synergique, **caractérisé par le fait que** ledit ingrédient actif végétal est un extrait naturel de châtaigne (*Castanea sativa*), avec du tanin comme substance constitutive la plus élevée, où ledit extrait naturel de châtaigne est obtenu par hydrolyse du bois de châtaignier (*Castanea sativa*) sans l'addition de substances étrangères ou synthétiques, où la teneur en tanin dans l'extrait naturel de châtaigne est d'environ 77 %, où ledit extrait naturel de châtaigne est contenu dans un taux pondéral entre 5 % et 50 % et lesdits moyens de support sont à base d'eau et comprennent des substances alcalines.

2. Suspension médicinale, **caractérisée par le fait qu'**elle comprend le composé médicinal selon la revendication 1, dans laquelle ledit extrait naturel de châtaigne est contenu dans un taux pondéral compris entre 5 % et 50 % et lesdits moyens de support sont à base d'eau et comprennent des réticulants.

3. Composé médicinal selon la revendication 1, destiné à être utilisé dans le traitement des inflammations, des maladies virales, des plaies, des brûlures et de l'ostéoporose.
